# EUROPEAN PATENT SPECIFICATION

(11) **EP 2 863 855 B1**
(45) Date of publication and mention of the grant of the patent: **25.09.2019**
(21) Application number: 13809171.5
(22) Date of filing: 30.05.2013
(51) Int. Cl.: A61F 13/15, A61F 13/02, A61K 9/70, A61F 13/00

(54) **MEDICAL DRESSING WITH MULTIPLE ADHESIVES**
MEDIZINISCHER VERBAND MIT MEHREREN HAFTSTOFFEN
PANSEMENT MÉDICAL À ADHÉSIFS MULTIPLES

(30) Priority: 26.06.2012 US 201261664246 P
(43) Date of publication of application: 29.04.2015
(73) Proprietor: 3M Innovative Properties Company, St. Paul, MN 55133-3427 (US)
(72) Inventor: DECABOOTER, Daniel P., Saint Paul, Minnesota 55133-3427 (US); HEINECKE, Steven B., Saint Paul, Minnesota 55133-3427 (US); HOLM, David R., Saint Paul, Minnesota 55133-3427 (US); JACOBSON, Richard L., Saint Paul, Minnesota 55133-3427 (US); LUNDQUIST, Kevin G., Saint Paul, Minnesota 55133-3427 (US); PETERSON, Donald G., Saint Paul, Minnesota 55133-3427 (US); HANSON, Jennifer N., Saint Paul, Minnesota 55133-3427 (US); TSE, Kiu-Yuen, Saint Paul, Minnesota 55133-3427 (US)
(74) Representative: Vossius & Partner Patentanwälte Rechtsanwälte mbB
(86) International application number: PCT/US2013/043319
(87) International publication number: WO 2014/003957

(56) References cited:
- WO-A1-2008/043364
- WO-A1-2012/033456
- WO-A1-2012/033456
- WO-A2-2008/082444
- US-A- 5 153 040
- US-A1- 2004 077 984
- US-A1- 2006 083 776
- US-A1- 2010 292 626
- US-B1- 6 791 003
- US-B1- 6 791 003

## Description

### Field

The present disclosure relates to a medical dressing with multiple adhesives. In particular, the present disclosure relates to a medical dressing for securing a medical device to skin, where a first adhesive secures to the medical device, while a gentle adhesive can secure to the skin.

### Background

Medical adhesive dressings are used for a variety of medical applications, such as for securing medical devices to skin. Adhesive dressings can provide barrier protection to a wound or insertion site from infectious species. Transparent adhesive dressings are commonly used at a catheter site because visual monitoring of the insertion site can be achieved without removing the dressing. Poor or weak securement of an inserted medical device and potential infiltration of antagonistic microbes into an insertion site can result in the need for catheter reinsertion or can lead to complications such as phlebitis and catheter related bloodstream infections (CRBSIs). When a patient experiences a CRBSI, mortality and morbidity rates increase significantly. Very strong adhesives used to secure an inserted medical device, which are also in contact with skin, can be very damaging to skin when removed. Use of a more gentle adhesive can significantly reduce skin damage, i.e., edema, erythema, skin cell stripping, after the adhesive is removed from the skin. However, common gentle adhesives are silicone-based adhesives that have poor adhesion to the inserted medical device. US 2004/0077984 A1 discloses a film dressing for use at catheter sites.

### Summary

The invention concerns the use of a medical dressing for securing a medical device to the skin and is defined as in the claims.

The disclosed medical dressing includes multiple adhesives. A first adhesive can strongly secure to the medical device, while a gentle adhesive secures to skin. Therefore, the medical device is secure, while the skin is in contact with a gentle adhesive. Selection of the adhesive, as well as the positioning of the adhesive on the medical dressing disclosed result in a medical dressing that effectively secures a medical device to a patient, while gently securing to skin.

In one embodiment, a medical dressing comprises a substrate having an upper surface and an outer contact surface, opposite the upper surface, a first adhesive having a first adhesion at a first portion of the outer contact surface, a second adhesive having a second adhesion at a second portion of the outer contact surface. The first adhesive has an adhesion greater than the second adhesive. The second portion with the second adhesive is adjacent a perimeter and extends along at least 25% of the perimeter of the substrate.

In one embodiment, a medical dressing comprises a substrate having an upper surface and an outer contact surface, opposite the upper surface, an acrylate adhesive at a first portion of the outer contact surface, a silicone adhesive at a second portion of the outer contact surface, wherein the second portion with the silicone adhesive is adjacent a perimeter and extends along at least 25% of the perimeter of the substrate.

In one embodiment, a method of securing a medical device comprises placing the medical device adjacent skin; applying a medical dressing, which comprises a substrate having an upper surface and an outer contact surface, opposite the upper surface, a first adhesive having a first adhesion at a first portion of the outer contact surface and a second adhesive having a second adhesion at a second portion of the outer contact surface, over the medical device such that the first adhesive in contact with the medical device and the second adhesive is in contact with the skin.

### Brief Description of the Drawings

FIG. 1 is a bottom view of a first embodiment of an adhesive dressing;
FIG. 2 is a side sectional view of the adhesive dressing shown in FIG. 1;
FIG. 3 is a top view of a another embodiment of an adhesive dressing applied over a medical device;
FIG. 4 is a bottom view of the adhesive dressing of FIG. 3;
FIG. 5 is a top view of another embodiment of an adhesive dressing;
FIG. 6 is a side sectional view of the embodiment of FIG. 5;
FIG. 7 is a top view of another embodiment of an adhesive dressing;
FIG. 8 is a side sectional view of the embodiment of FIG. 7;
FIG. 9 is a top view of another embodiment of an adhesive dressing;
FIG. 10 is a side sectional view of the embodiment of the adhesive dressing shown in FIG. 9 applied over a medical device.

While the above-identified drawings and figures set forth embodiments of the invention, other embodiments are also contemplated, as noted in the discussion. In all cases, this disclosure presents the invention by way of representation and not. The figures may not be drawn to scale.

### Detailed Description

Disclosed is a medical adhesive dressing. The adhesive dressing includes a substrate having an upper surface and an outer contact surface. The outer contact surface contains at least two adhesives each on a portion of the outer contact surface of the substrate. The outer contact surface is the surface of the adhesive dressing that is intended to come in contact with skin. In some embodiments, the outer contact surface can come in contact with skin while also securing a medical device to the skin. Generally, when also securing a medical device, the adhesive dressing covers and surrounds at least part of the device, while the outer edges at the perimeter of the adhesive dressing contact the skin.

### Substrate

The substrate can be a single layer of material or may be comprised of two or more layers. The substrate is typically a flexible material. The substrate may be a film, paper, woven, knitted, or nonwoven material or a combination of one or more layers of film, paper, woven, knitted, or nonwoven. In some embodiments, a transparent substrate is desirable to allow for viewing of the underlying skin or medical device.

In one embodiment, the substrate has high moisture vapor permeability, but generally impermeable to liquid water so that microbes and other contaminants are sealed out from the area under the substrate. One example of a suitable material is a high moisture vapor permeable film such as described in US Patents 3,645,835 and 4,595,001. In high moisture vapor permeable film/adhesive composites, the composite should transmit moisture vapor at a rate equal to or greater than human skin such as, for example, at a rate of at least 300 g/m² /24 hrs at 37°C/100-10% RH, or at least 700 g/m² /24 hrs at 37°C/100-10% RH, or at least 2000 g/m² /24 hrs at 37°C/100-10% RH using the inverted cup method as described in U.S. Pat. No. 4,595,001. Perforated substrates or films or pattern coated adhesives may be used to increase the moisture vapor transmission. In one embodiment, the substrate is an elastomeric polyurethane, polyester, or polyether block amide films. These films combine the desirable properties of resiliency, high moisture vapor permeability, and transparency.

To add strength to a very thin film, all or a portion of the film may include an additional layer of another film, woven, knitted, or nonwoven fabric. For example, a 3M Tegaderm I.V. Advanced Dressing, available from 3M Company, includes a portion of the substrate which additionally includes a nonwoven substrate secured to a thin, transparent film.

### Adhesives

Adhesive means a material that can adhere the substrate to skin. In one embodiment, the adhesive is a pressure sensitive adhesive that can adhere the substrate to skin with application of light pressure and without the need for heat or other external sources to active adhesion.

In one embodiment, the first adhesive at the first portion of the outer contact surface of the substrate has an adhesion that is higher than the second adhesive at the second portion of the outer contact surface. In one embodiment, the first and second adhesive may be of the same or similar classes of adhesive, but have different adhesions. For example, changes in adhesive composition, adhesive thickness, or adhesive surface area can change the adhesion. The first adhesive is an acrylate-containing adhesive.

"Adhesion" means the force required to separate an adhesive from the substrate. Adhesion can be measured in a number of ways. For example, adhesion can be defined by peel force or shear force. For example, adhesion can be defined by peel adhesion using ASTM D3330/D3330M-04(2010). For example, adhesion can be defined by shear adhesion using ASTM D3654M-06(2011). Adhesion is highly dependent on the specific substrate being adhered to, as well as the time the PSA is allowed to dwell on the substrate.

For example, typical peel adhesion values exhibited by pressure sensitive adhesives in medical dressings maybe in the range of 20 to 300 g/cm as measured from stainless steel. In one embodiment, at least 10% higher peel adhesion, as measured by ASTM D3330 / D3330M - 04(2010), of the first adhesive over the second adhesive may realize the benefit of both securing to the medical device, while providing gentle adhesion to the skin.

The first adhesive is an acrylate adhesive and the second adhesive is a silicone adhesive. The term "acrylate" or "acrylate-based" as used herein refers to monomeric acrylic or methacrylic esters of alcohols. Acrylate and methacrylate monomers are referred to collectively herein as "acrylate" monomers. Materials that are described as "acrylate-based" contain at least some acrylate monomers and may contain additional co-monomers.

Acrylate adhesives are well suited for securing adhesive dressings to medical devices or skin. The adhesion can be manipulated to have high adhesion or low adhesion. Generally, the adhesion between acrylate adhesives and another material will increase over time. This property makes acrylate adhesives well suited as the first adhesive, which is intended to secure a medical device.

Suitable acrylate adhesives that can be applied to skin such as the acrylate copolymers are described in U.S. Patent No. RE 24,906, the disclosure of which is hereby incorporated by reference. In particular, a 97:3 iso-octyl acrylate:acrylamide copolymer. Another acrylate adhesive is an 70:15:15 isooctyl acrylate: ethyleneoxide acrylate:acrylic acid terpolymer, as described in U.S. Pat. No. 4,737,410 (Example 31), the disclosure of which is hereby incorporated by reference. Other useful acrylate adhesives are described in U.S. Pat. Nos. 3,389,827, 4,112,213, 4,310,509, and 4,323,557.

The term "silicone" or "silicone-based" as used herein refers to polymers that contain units with dialkyl or diaryl siloxane (-SiR₂O-) repeating units. The silicone-based polymers may be segmented copolymers or polysiloxanes polymers. The terms silicone and siloxane are used interchangeably.

Generally, silicone adhesives are able to effectively secure dressings and tape to skin and upon removal from the skin product little or no skin damage. Typically, the silicone adhesives do not adhere well to polymer-based substrates, like tubing or hardgoods. The gentle removal of silicone adhesives from skin make silicone adhesives well suited as the second adhesive, which is intended to contact the skin.

An example of a suitable silicone adhesive is disclosed in PCT Publications WO2010/056541 and WO2010/056543. A radiation cured silicone adhesive is particularly well suited for this application because the extent of crosslinking, and therefore adhesion of the silicone adhesive can be better controlled. Other examples of silicone gel adhesives systems include products marketed with the trade names: Dow Corning MG 7-9850, Wacker SILPURAN® 2110 and 2130, Bluestar SILBIONE® RT Gel 4317 and 4320, Nusil MED-6345 and 6350.

For adhesive in contact with skin, it is desirable that the adhesive is able to transmit moisture vapor at a rate greater to or equal to that of human skin. While such a characteristic can be achieved through the selection of an appropriate adhesive, it is also contemplated that other methods of achieving a high relative rate of moisture vapor transmission may be used, such as perforating the adhesive or pattern coating the adhesive, as described in U.S. Pat. No. 4,595,001 and U.S. Pat. App. Pub. 2008-0233348. The first or second adhesive can be applied in a discontinuous manner to form the first or second portion, respectively.

### Arrangement of the adhesive

The first adhesive is located at a first portion of the outer contact surface. The second adhesive is located at a second portion of the outer contact surface. The first portion is an area on the outer contact surface that is separate from the second portion that is an area on the outer contact surface. In one embodiment, there is more than one first portion having the first adhesive. In one embodiment, there is more than one second portion having the second adhesive.

The first portion being either the higher adhesion adhesive or an acrylate adhesive is intended to secure to the medical device and the contact with skin is minimized. The second portion being either the lower adhesion or a silicone adhesive is intended to secure to skin. To most effectively secure the adhesive dressing to the skin, while also securing the medical device, the perimeter of the substrate of the adhesive dressing should contact skin. Therefore, the second portion, containing the second adhesive, should be at the perimeter of the adhesive dressing.

The second portion extends along at least 25% of the perimeter of the substrate. In another embodiment, the second portion extends along at least 50% of the perimeter of the substrate.

Depending on the arrangement of the adhesive dressing, the type of medical device, it may be desirable to have the first portion along the perimeter. However, typically the first portion will extend along less than 50% of the perimeter of the substrate. In another embodiment, the first portion extends along less than 25% of the perimeter of the substrate. In another embodiment, the second portion extends along the entire perimeter of the substrate and surrounds the first portion.

It may be desirable to have the total area of the first portion having the first adhesive be less than the total area of the second portion having the second adhesive. In one embodiment, the area of the first portion comprises less than 50% of the area of the outer contact surface.

An optional third portion that is essentially free of adhesive may also be included on the outer contact surface. An adhesive-free third portion may be desirable to coincide with the area directly over the insertion site of a medical device, over a wound, or over a portion of a medical device where adhesion between the outer contact surface and the medical device is undesirable. This adhesive-free third portion may comprise an absorbent material, such as gauze or foam.

The adhesive dressing is not limited to including only the first and second adhesives but may include additional portions with additional adhesives.

### Optional Components

An optional release liners may be included that covers all or a portion of the adhesives to prevent contamination of the adhesives. In one embodiment, the package that contains the adhesive dressing may serve as a release liner. Suitable release liners can be made of kraft papers, polyethylene, polypropylene, polyester or composites of any of these materials. In one embodiment, the liners are coated with release agents such as fluorochemicals or silicones. For example, U.S. Pat. No. 4,472,480, describes low surface energy perfluorochemical liners. In one embodiment, the liners are papers, polyolefin films, or polyester films coated with silicone release materials.

An optional carrier may be included that covers all or a portion of the upper surface of the substrate, providing structural support if the material of the substrate is thin and highly flexible. The carrier maybe removable from the upper surface once the adhesive dressing is placed on skin. The carrier can be constructed of a variety of materials such as fabric that are woven or kitted, nonwoven material, papers, or film. In one embodiment, the carrier is along the perimeter of the upper surface of the substrate and is removable from the upper surface, similar to the carrier used the 3M Tegaderm™ Transparent Film Dressing, available from 3M Company, St. Paul, MN.

An optional antimicrobial component may be included that is either separate from the adhesive dressing or may be integral with the dressing. The antimicrobial component is placed near or adjacent to the insertion site of the medical device to inhibit microbial growth in and around the insertion site. The antimicrobial component can be absorbent foam or gel, such as used in a 3M Tegaderm™ CHG I.V. Securement Dressing, available from 3M Company.

### Use of the Medical Adhesive Dressing

The adhesive dressing is secured to skin. To minimize potential damage to skin, the second adhesive is placed in contact with skin. The first adhesive can contact skin, but typically the first adhesive is placed in contact with a medical device that is to be secured to skin. Therefore, the adhesive dressing overlies the medical device and secures to the skin around at least a portion of the medical device.

Various medical devices can be placed adjacent to skin. Some include a portion that is inserted into the skin at an insertion site. Adhesive dressings placed over medical devices that are inserted in to the skin provide three advantages. First, if the adhesive dressing is transparent, a health care provided can view the insertion site and see if the device is properly inserted and can look for signs of infection. Second, the adhesive dressing can cover and seal the insertion site to keep contaminants out of the body and reduce infection. Third, the adhesive dressing can secure the medical dressing so that it is not dislodged, which would require accessing the insertion site and further expositing the site to potential pathogens for infection.

Various medical devices are held near a patient, but not necessarily including a portion that inserts into skin. For examples drapes, tubes, heart monitor, sensors for moisture and temperature may be near a patient but necessarily inserted into the skin or body. Examples of medical devices that include a portion inserted into skin are catheters, pumps, ports, such as those containing a Huber needle inserted into a body that should secured or covered. The medical device may include various components such as, for example, a needle, a needle hub, or tubing to which the first adhesive secures.

In one embodiment, a kit is provided that includes the medical device and the adhesive dressing. The kit may include other optional components that may be desirable for placement or securement of the medical device such as, for example, gloves, antiseptic preparation for cleaning skin prior to application of the medical dressing, skin protectant materials, gauze, tape, tubing, antimicrobial patch for placement at the insertion site, or additional securement devices.

Wound dressings have been created that include one or more adhesives on a contact surface. However, those types of dressings are of a construction completely opposite to the adhesive dressing disclosed herein. Wound dressing typically include the less aggressive adhesive at the center portion, which would come in contact with the broken skin of the wound and the more aggressive adhesive at the perimeter to keep the dressing in place. Here, most of the perimeter, which then comes in contact with the skin, is covered with the less aggressive adhesive. Also, the more aggressive adhesive is only at portion of the perimeter, if at all.

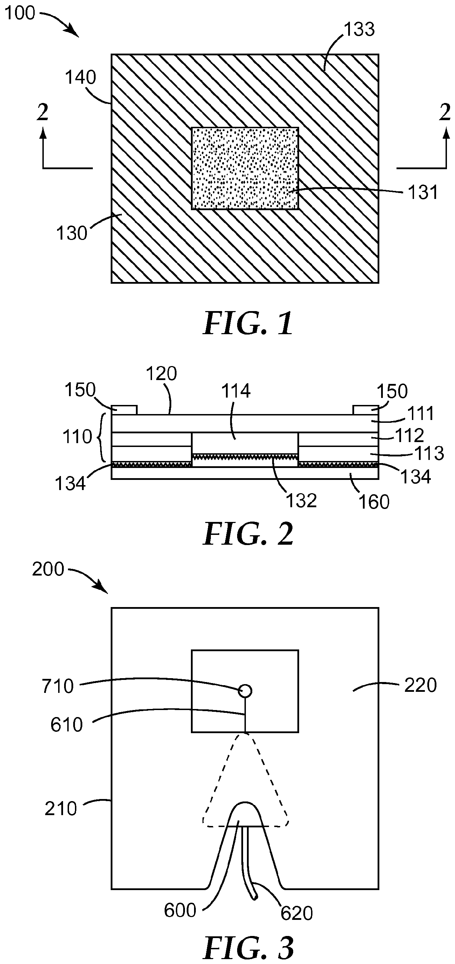

Specific embodiments of the adhesive dressing are shown in FIGS. 1-10 and described below. It is understood, that the various arrangements of the components and materials from one embodiment could be interchangeable with components and materials described in the other embodiment. For example, for simplicity FIGS. 1 and 2 show various elements of an adhesive dressing and includes a carrier and release layer. FIG. 3 does not show an adhesive dressing with a carrier and release layer, but instead shows the medical dressing applied over skin and a medical device. It is understood, that each of the adhesive dressings may include carrier and release layers and may be applied over skin and a medical device. Additionally, the various embodiment show various arrangements of substrate layers and various placements of the first portion and second portions containing the adhesive. It is understood that any arrangement of substrate configurations could be used in combination with any of the disclosed arrangements of adhesives. Where applicable, the first adhesive at the first portion is shown in stippling, and the second adhesive at the second portion is shown in cross-hatching.

FIG. 1 is a bottom view of a first embodiment of an adhesive dressing 100. FIG. 2 is a side sectional view of the embodiment shown in FIG. 1. The adhesive dressing 100 comprises a substrate 110 having an upper surface 120 and an outer contact surface 130. The outer contact surface 130 includes a first portion 131, which contains the first adhesive 132, and a second portion 133, which contains the second adhesive 134. The adhesive dressing 100 comprises a perimeter 140 that is the outer edge portions of the adhesive dressing 100.

The substrate comprises all of the structural layers between the exposed adhesive 132, 134 and the upper surface 120. In this embodiment, the substrate 110 comprises a plurality of layers. In this embodiment, the first layer 111 forms the upper surface 120 and extends continuously over the adhesive dressing 100. The second layer 112 extends adjacent the perimeter 140 but is not located in the first portion 131, which contains the first adhesive 132. The second layer 112 provides structural strength to the adhesive dressing 100 and in this embodiment, comprises a nonwoven. Generally, layers are included to aid in adhesion. For example, third layer 113 is included to aid in adhesion of the first adhesive 133 and fourth layer 114 is included to aid in adhesion of second adhesive 134.

At the outer contact surface 130, a first portion 131 includes the first adhesive 132. A second portion 133 that is separate from the first portion 131 includes the second adhesive 134. In this embodiment, the entire perimeter 140 comprises the second portion 133 containing the second adhesive. The first portion 131 containing the first adhesive 132 is entirely surrounded by the second portion.

Overall, the substrate 110 remains a thin, flexible and water vapor permeable material. Therefore, optional carrier layer 150 is included on the upper surface 120 to provide strength to the substrate 110. Carrier layer 150 typically is removable after the adhesive dressing 110 is applied to skin. An optional release layer 160 is included to cover the outer contact surface 130 and prevent contamination of the first and second adhesive 132, 134.

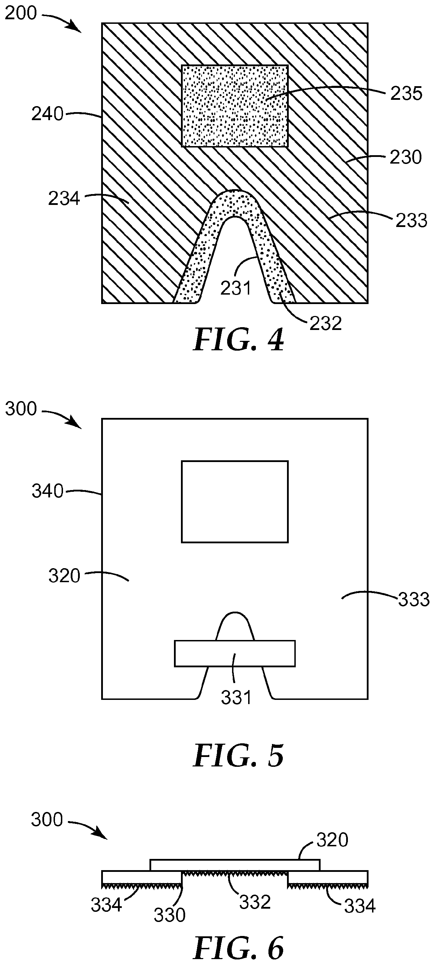

FIG. 3 is a top view of another embodiment of an adhesive dressing 200 applied over skin and a medical device 600. FIG. 4 is a bottom view of the adhesive dressing 200. The adhesive dressing 200 comprises a substrate 210 having an upper surface 220 and an outer contact surface 230. As shown in the bottom view, FIG. 4, the outer contact surface 230 contains the first adhesive 232 and second adhesive 234. The adhesive dressing 200 comprises a perimeter 240 that is the outer edge portions of the adhesive dressing 200.

The substrate 210 may comprise one or more layers as previously described. In this embodiment, it is desirable the insertion site 710 is visible and therefore the substrate 210 over the insertion site 710 comprises a transparent material. Underlying the upper surface 220, at the outer contact surface 230, a first portion 231 includes the first adhesive 232. A second portion 233 that is separate from the first portion 231 includes the second adhesive 234. In this embodiment, the perimeter 240 comprises the second portion 233 containing the second adhesive 234 and the first portion 231 containing the first adhesive 232. The first portion 231 containing the first adhesive 232 is positioned adjacent to where the medical device 600 would extend outwardly from the insertion site 710 to overly the medical device 600. It also may be desirable that a third portion 235 is formed, in this case over the insertion site 710, that is free of adhesive such that upon removal of the adhesive dressing 200 the insertion needle 610 of the medical device 600 at the insertion site 710 is not pulled away from the skin. This adhesive dressing 200 may also include optional carrier layers or release layers, which are not shown.

FIG. 5 is a top view of another embodiment of an adhesive dressing 300. FIG. 6 is a side sectional view of the embodiment of FIG. 5 through the first portion 331, which is shown as a tape strip. The adhesive dressing 300 shown in FIGS. 5 and 6 is similar in shape to that shown in FIGS. 3 and 4. However, in this embodiment, the first portion 331 containing the first adhesive 332 is a tape strip that is applied over a the upper surface 320 of the adhesive dressing 300 such that a first portion 331 containing the first adhesive 332 is exposed at outer contact surface 330. The tape strip may be permanently secured to the upper surface 320 once applied or may be removable from the upper surface 320.

In this embodiment, the perimeter 340 comprises the second portion 333 containing the second adhesive 334 and the first portion 331 containing the first adhesive 332. The first portion 331 containing the first adhesive 332 is formed from the tape strip and is positioned adjacent to where the medical device (not shown) would extend outwardly from the insertion site. It is understood that in this embodiment, any number of substrate layers can be included, that a medical device similar to that shown in FIGS. 3 and 4 should be secured to skin, and that optional carrier layer and release layer could be included.

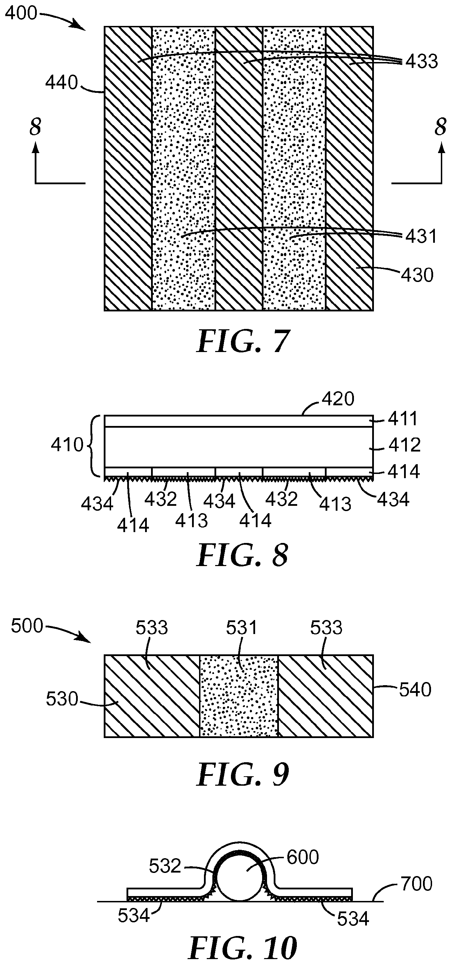

FIG. 7 is a top view of another embodiment of an adhesive dressing 400. FIG. 8 is a side sectional view of the embodiment of FIG. 7. The adhesive dressing 400 comprises a substrate 410 having an upper surface 420 and an outer contact surface 430, which contains the first adhesive 432 and second adhesive 434. The adhesive dressing 400 comprises a perimeter 440 that is the outer edge portions of the adhesive dressing.

The substrate 410 comprises a plurality of layers. In this embodiment, the first layer 411 forms the upper surface 420 and extends continuously over the adhesive dressing 400. The second layer 412 is an absorbent foam, which makes this dressing well suited as a wound dressing. A third layer 413 is included to aid in adhesion of the first adhesive 433 and fourth layer 414 is included to aid in adhesion of second adhesive 434.

At the outer contact surface 430, a first portion 431 includes the first adhesive 432. A second portion 433 that is separate from the first portion 431 includes the second adhesive 434. In this embodiment, the first portion 431 and second portion 433 each comprise linear sections, wherein the first portion 431 forms most of the perimeter 440. This arrangement helps combine the benefits of gentle adhesives one the skin while also including a more aggressive adhesive for securing a medical device, or to help in securing the adhesive dressing 400 to skin.

FIG. 9 is a top view of another embodiment of an adhesive dressing 500. FIG. 10 is a side sectional view of the adhesive dressing 500 applied over a medical device 600 and secured to skin 700. This embodiment is similar to that shown in FIGS. 7 and 8 except that there is a single first portion 531 and at the ends of the adhesive dressing 500 is each a second portion 533. Although, somewhat similar in placement of the first and second portions 531, 533, it is understood that any number of substrates may be included. For example, the adhesive dressing 500 shown in FIG. 8 may be a tape strip wherein the substrate is a single or multilayer, transparent or opaque film, woven, knitted, or nonwoven material.

Although specific embodiments of this invention have been shown and described herein, it is understood that these embodiments are merely illustrative of the many possible specific arrangements that can be devised in application of the principles of the invention. Numerous and varied other arrangements can be devised in accordance with these principles by those of ordinary skill in the art without departing from the spirit and scope of the invention. Thus, the scope of the present invention should not be limited to the structures described in this application, but only by the structures described by the language of the claims and the equivalents of those structures.

## Claims

1. Use of a medical dressing for securing a medical device to skin, the medical dressing comprising:
a substrate having an upper surface and an outer contact surface, opposite the upper surface;
a first adhesive having a first adhesion at a first portion of the outer contact surface, wherein the first adhesive is an acrylate-containing adhesive;
a second adhesive having a second adhesion at a second portion of the outer contact surface, wherein the second adhesive is a silicone-containing adhesive;
wherein the first adhesive has an adhesion greater than the second adhesive;
wherein the second portion with the second adhesive is adjacent a perimeter and extends along at least 25% of the perimeter of the substrate; and
wherein the first adhesive is placed in contact with the medical device that is to be secured to the skin.

2. The use of the medical dressing of any one of the preceding claims, wherein the substrate is a composite of one or more materials secured to one another.

3. The use of the medical dressing of any one of the preceding claims, wherein the substrate comprises a transparent, moisture vapor permeable polymeric film.

4. The use of the medical dressing of any one of the preceding claims, wherein the first portion extends along at least 50% of the perimeter.

5. The use of the medical dressing of any one of the preceding claims, wherein the first portion is entirely surrounded by the second portion.

6. The use of the medical dressing of any one of the preceding claims, comprising a plurality of first portions containing the first adhesive.

7. The use of the medical dressing of any one of the preceding claims, comprising a plurality of second portions containing the second adhesive.

8. The use of the medical dressing of any one of the preceding claims, further comprising a third portion of the outer contact surface that is essentially free of adhesive.

9. The use of the medical dressing of any one of the preceding claims, wherein the first portion comprises a tape strip.

## Patentansprüche

1. Verwendung eines medizinischen Verbandes zum Befestigen einer medizinischen Vorrichtung an Haut, wobei der medizinische Verband Folgendes aufweist:
ein Substrat mit einer oberen Oberfläche und einer äußeren Kontaktoberfläche gegenüber der oberen Oberfläche;
einen ersten Klebstoff mit einer ersten Haftfähigkeit an einem ersten Abschnitt der äußeren Kontaktoberfläche, wobei der erste Klebstoff ein acrylathaltiger Klebstoff ist;
einen zweiten Klebstoff mit einer zweiten Haftfähigkeit an einem zweiten Abschnitt der äußeren Kontaktoberfläche, wobei der zweite Klebstoff ein silikonhaltiger Klebstoff ist;
wobei der erste Klebstoff eine höhere Haftfähigkeit als der zweite Klebstoff aufweist;
wobei der zweite Abschnitt mit dem zweiten Klebstoff an einen Umfang angrenzt und sich entlang mindestens 25 % des Umfangs des Substrats erstreckt; und
wobei der erste Klebstoff in Kontakt mit der medizinischen Vorrichtung positioniert wird, die an der Haut befestigt werden soll.

2. Verwendung des medizinischen Verbandes nach einem der vorstehenden Ansprüche, wobei das Substrat ein Verbundstoff aus einem oder mehreren aneinander befestigten Materialien ist.

3. Verwendung des medizinischen Verbandes nach einem der vorstehenden Ansprüche, wobei das Substrat eine transparente, feuchtigkeitsdampfdurchlässige Polymerfolie aufweist.

4. Verwendung des medizinischen Verbandes nach einem der vorstehenden Ansprüche, wobei sich der erste Abschnitt entlang mindestens 50 % des Umfangs erstreckt.

5. Verwendung des medizinischen Verbandes nach einem der vorstehenden Ansprüche, wobei der erste Abschnitt vollständig von dem zweiten Abschnitt umgeben ist.

6. Verwendung des medizinischen Verbandes nach einem der vorstehenden Ansprüche, aufweisend mehrere erste Abschnitte, die den ersten Klebstoff enthalten.

7. Verwendung des medizinischen Verbandes nach einem der vorstehenden Ansprüche, aufweisend mehrere zweite Abschnitte, die den zweiten Klebstoff enthalten.

8. Verwendung des medizinischen Verbandes nach einem der vorstehenden Ansprüche, ferner aufweisend einen dritten Abschnitt der äußeren Kontaktoberfläche, der im Wesentlichen frei von Klebstoff ist.

9. Verwendung des medizinischen Verbandes nach einem der vorstehenden Ansprüche, wobei der erste Abschnitt einen Klebestreifen aufweist.

## Revendications

1. Utilisation d'un pansement médical pour fixer un dispositif médical sur la peau, le pansement médical comprenant :
un substrat ayant une surface supérieure et une surface de contact externe, opposée à la surface supérieure ;
un premier adhésif ayant une première adhérence au niveau d'une première partie de la surface de contact externe, dans laquelle le premier adhésif est un adhésif contenant un acrylate ;
un deuxième adhésif ayant une deuxième adhérence au niveau d'une deuxième partie de la surface de contact externe, dans laquelle le deuxième adhésif est un adhésif contenant une silicone ;
dans laquelle le premier adhésif a une adhérence supérieure à celle du deuxième adhésif ;
dans laquelle la deuxième partie avec le deuxième adhésif est adjacente à un périmètre et s'étend le long d'au moins 25 % du périmètre du substrat ; et
dans laquelle le premier adhésif est placé en contact avec le dispositif médical destiné à être fixé sur la peau.

2. Utilisation du pansement médical selon l'une quelconque des revendications précédentes, dans laquelle le substrat est un composite d'un ou plusieurs matériaux fixés les uns sur les autres.

3. Utilisation du pansement médical selon l'une quelconque des revendications précédentes, dans laquelle le substrat comprend un film polymère transparent, perméable à la vapeur d'humidité.

4. Utilisation du pansement médical selon l'une quelconque des revendications précédentes, dans laquelle la première partie s'étend le long d'au moins 50 % du périmètre.

5. Utilisation du pansement médical selon l'une quelconque des revendications précédentes, dans laquelle la première partie est entièrement entourée par la deuxième partie.

6. Utilisation du pansement médical selon l'une quelconque des revendications précédentes, comprenant une pluralité de premières parties contenant le premier adhésif.

7. Utilisation du pansement médical selon l'une quelconque des revendications précédentes, comprenant une pluralité de deuxièmes parties contenant le deuxième adhésif.

8. Utilisation du pansement médical selon l'une quelconque des revendications précédentes, comprenant en outre une troisième partie de la surface de contact externe qui est essentiellement exempte d'adhésif.

9. Utilisation du pansement médical selon l'une quelconque des revendications précédentes, dans laquelle la première partie comprend une bande de ruban.
